# EUROPEAN PATENT SPECIFICATION

(11) **EP 2 370 036 B1**
(45) Date of publication and mention of the grant of the patent: **19.02.2014**
(21) Application number: 09771713.6
(22) Date of filing: 03.12.2009
(51) Int. Cl.: A61F 13/00, A61F 17/00

(54) **ABSORBENT MEDICAL BODY, IN PARTICULAR FOR REMOVING WOUND FLUIDS FROM HUMAN AND/OR ANIMAL BODY CAVITIES**
SAUGFÄHIGER MEDIZINISCHER KÖRPER, INSBESONDERE ZUM ENTFERNEN VON WUNDFLÜSSIGKEITEN AUS MENSCHLICHEN UND/ODER TIERKÖRPERKAVITÄTEN
CORPS MÉDICAL ABSORBANT, EN PARTICULIER POUR EXTRAIRE LES FLUIDES DE BLESSURE DES CAVITÉS CORPORELLES HUMAINES ET/OU ANIMALES

(30) Priority: 03.12.2008 DE 102008061536
(43) Date of publication of application: 05.10.2011
(73) Proprietor: Aesculap AG, 78532 Tuttlingen/Donau (DE)
(72) Inventor: ODERMATT, Erich, CH-8200 Schaffhausen (CH); BERNDT, Ingo, 52066 Aachen (DE); KÖNIG, Silke, 78628 Rottweil (DE)
(74) Representative: Patentanwälte Ruff, Wilhelm, Beier, Dauster & Partner
(86) International application number: PCT/EP2009/008625
(87) International publication number: WO 2010/063466

(56) References cited:
- EP-A1- 1 820 395
- EP-A2- 1 649 844
- US-A- 5 395 309
- US-A- 6 123 697
- US-A1- 2005 137 512
- US-A1- 2006 280 900
- US-A1- 2008 200 895

## Description

The invention relates to an absorbent medical sponge body, which is suitable primarily for removing wound fluids from human and/or animal body cavities.

One possible way of treating infectious body cavities is provided by so-called endoluminal vacuum therapy. This form of therapy employs a special wound drainage system which is composed essentially of an open-pore sponge for absorbing wound secretions and a drainage tube for removing or discharging the absorbed wound secretions. Such a wound drainage system is marketed by B. Braun Aesculap AG under the name Endo-SPONGE. For example, such a wound drainage system is used for the therapy of anastomotic insufficiencies in the rectum.

In general, the sponge is placed in the relevant body cavity by means of an overtube. The sponge is pressed together or compressed by the overtube, so that the sponge can be placed in the relevant body cavity. The overtube is conventionally produced by the injection moulding method and usually consists of a silicone material with an embedded metal spiral. The metal spiral provides the kink and cross-sectional stability when the overtube is bent. Production of the overtube is relatively cost-intensive. Furthermore, the technical opportunities are currently exhausted with a length of about 30 cm. However, possible applications for the previously described wound drainage system may be envisaged in which the sponge must be placed in a body cavity over a much longer guide length.

Longer guide lengths are required for example when treating aneurysms in the oesophagus, which may occur as a result of partial oesophageal resection. Such a partial resection is conventionally carried out in order to remove an oesophageal carcinoma. After the partial resection, either the stomach is raised and sutured to the shortened oesophagus, or an intestine portion is sutured between them. Protuberances may then occur in the region of the suturing, which can become larger owing to consecutively occurring infections.

Another problem is that the sponge unfolds again immediately after emerging from the overtube and normally can no longer be repositioned or re-placed inside the body cavity. Since even with a preliminary endoscopic study of the relevant body cavity, there is not always a guarantee that the sponge can be brought into the desired position inside the body cavity at the first attempt, a repositionably designed sponge would be of great medical benefit.

US 6,123,697 discloses a medical device comprising a packing member and a wrap member for absorbing fluid while applying pressure. US 2008/0200895 A1 discloses a catamenial tampon comprising a compressed absorbent member and an overwrap covering at least a portion of the exterior surface of the absorbent member.

US 2006/0280900 A1 relates to a water-absorbent structure comprising a panel-shaped assembly of synthetic fibres assembled in a form of a honeycomb construction. EP 1 649 844 A2 pertains to a sanitary tampon comprising an absorbent wrapped by a liquid-pervious sheet. US 2005/0137512 A1 refers to advanced haemorrhage control wound dressings which can be wrapped by a closed plastic film.

It is therefore an object of the present invention to provide an absorbent medical pad for removing wound fluids from infectious body cavities, which avoids the said disadvantages in the prior art. It should in particular be possible to place the absorbent body in a body cavity without an overtube and, if need be, re-place it inside the body cavity.

This object is achieved according to the invention by an absorbent medical sponge body for removing wound fluids from human and/or animal body cavities, comprising a film for the absorbent sponge body, wherein the film holds the absorbent sponge body together in a compressed form.

The invention provides an absorbent medical sponge body, or a medical product based on an absorbent sponge body and a film or sheath for the absorbent sponge body. The absorbent sponge body is contained in the film and is held together in a compressed form by it. In other words, owing to the film, the absorbent sponge body is provided in a compressed form. In this form, the absorbent sponge body can be placed in a body cavity comprising wound secretions or wound fluids. After optimal placement or positioning of the absorbent sponge body in the body cavity, the film is removed so that the absorbent sponge body can unfold or expand again. In the following, the terms "absorbent medical sponge body" and "absorbent sponge body" are used interchangeably.

As already mentioned, the absorbent sponge body according to the invention is suitable for removing wound fluids or wound secretions, i.e. pathological fluid accumulations, from human and/or animal body cavities. The absorbent sponge body is preferably used in the scope of so-called endoluminal vacuum therapy which will be described in more detail below. The absorbent sponge body according to the invention may, for example, be used for treating intra-abdominal or intracavity abscesses, fistulas, pancreatitis or the like. Regarding the fistulas, the absorbent medical sponge body may be employed for the treatment of fistulas of small intestine and/or gall bladder.

The body cavities in the context of the invention are generally so-called wound cavities. This is intended to mean naturally occurring or pathologically induced protuberances, generally of hollow organs, like blood vessels, large intestine, small intestine, gall bladder, esophagus, urethra etc.. For example, said protuberances may be present as diverticals, aneurysms, fistulas and/or abscesses within the body of a patient. The protuberances may become filled with wound secretions. The pathologically induced protuberances are often the result of operational interventions, in particular anastomoses. Protuberances may be formed there in the ligature or suture region, and may rapidly become larger owing to infection. Protuberances in the region of the suturing are often also referred to as so-called insufficiency cavities.

The wound fluids in the context of the invention are pathological fluids, in particular wound secretions, exudates, abscess fluids or intestinal contents.

Drainage in the context of the present invention is intended to mean the removal or discharge of wound fluids from human and/or animal body cavities.

In a preferred embodiment, the film comprises means for removing it, in particular for opening it. The film preferably comprises at least one perforation, preferably a plurality of perforations. In particular, the film which holds the absorbent sponge body together in a compressed form comprises a perforation line along which the film can be opened, in particular by tearing, under tensile stress and can therefore be removed from the relevant body cavity.

The film itself is preferably formed from a liquid-impermeable material. In other words, the film preferably represents a form of artificial barrier for wound secretions that have accumulated in body cavities, so that premature unfolding or expansion or deployment of the absorbent sponge body in a body cavity can be avoided.

In another embodiment, the film is formed from a polymer, in particular a synthetic polymer. The polymer may be a homo or a copolymer. Copolymers in the context of the present invention are polymers that are composed of at least two different monomer units. The film is preferably formed from a non-absorbable polymer, in particular selected from the group consisting of silicone, silicone rubber, polypropylene, polyethylene, polyester, polyamides, derivatives thereof, copolymers thereof and mixtures, in particular blends, thereof. According to the invention, however, it is also possible in principle for the film to be formed from an absorbable polymer, for example selected from the group consisting of polylactide, polyglycolide, poly-ε-caprolactone, polytrimethylene carbonate, poly-para-dioxanone, copolymers thereof and mixtures, in particular blends, thereof.

Preferably, the film is formed as a tubular film (foil) or a blown film (foil). The film may have a thickness of between 0.05 and 0.5 mm, in particular 0.1 and 0.2 mm.

In another embodiment, the film per se is provided as a hollow body, in particular a sleeve or tube. Particularly preferably, the hollow body comprises a lumen which contracts under tensile stress in the longitudinal direction of the hollow body and is enlarged under compression in the longitudinal direction of the hollow body.

In a further embodiment, the absorbent sponge body comprises a lubricant that is present between the absorbent sponge body and the film. The lubricant advantageously facilitates a more convenient removal, in particular taking off, of the film after the placement of the absorbent sponge body. The lubricant itself may be present in a dry or wet form. For instance, it is within the scope of the present invention that the lubricant is present in a dry form and may be wetted or rehydrated subsequent to the placement of the absorbent sponge body in a human and/or animal body cavity. The wetting or rehydration of the lubricant may be realized by the aid of a flushing tube.

In a further embodiment, the film is oversized in relation to the absorbent sponge body. Preferably, the film is oversized at its distal end (away from the body of a patient). Such an oversizing makes it easy for a surgeon to remove, in particular take off, the film after the placement of the absorbent sponge body in a human and/or animal body cavity. In order to facilitate the removal of the film in this case, the film generally comprises an open proximal end (near the body of a patient). Furthermore, it is generally within the scope of the present invention that the film is oversized at its proximal end. For instance, the film may be folded there in order to form a pointed distal end that advantageously facilitates a more convenient intrusion of the absorbent sponge body into a human and/or animal body cavity. After the placement of the absorbent sponge body the film may be removed thereby unfolding the film at its proximal end and thereby generating an opening at the proximal end that facilitates the removal of the film.

In a preferred embodiment, the absorbent sponge body in the compressed form has a volume reduced by from 10 to 90%, in particular from 20 to 80%, preferably from 40 to 70%, than without the film. The absorbent sponge body is preferably provided compressed in two dimensions. For example, the absorbent sponge body may be provided in a radially compressed form.

The compressed absorbent sponge body may in principle be provided in various shapes. For instance, starting from a basic shape, the absorbent sponge body may be adapted in shape and size, in particular cut, for a body cavity to be treated. For example, the absorbent sponge body may have a circular, oval, triangular, square, trapezoidal, rhomboid, pentagonal or five-sided, hexagonal, star- or cross-shaped cross section. According to the invention, the absorbent sponge body may also be formed as a hollow body, for example as a tube, pipe or hollow cylinder.

The absorbent sponge body is preferably designed to be porous, particularly preferably open-porous or open-pored. The absorbent sponge body may in principle have a pore size of between 100 and 1500 µm, in particular 200 and 1000 µm, preferably 400 and 800 µm, more preferably 400 µm to 600 µm.

In another embodiment, the absorbent sponge body comprises active agents, in particular selected from the group consisting of antimicrobial, antiseptic, disinfectant, growth-promoting, odour-inhibiting and anti-inflammatory active agents. For example triclosan, polyhexamethylene biguanide (PHMB), copper, zinc, silver, compounds thereof, salts thereof may be mentioned as antimicrobial active agents. The active agents may generally be provided in particulate form, particularly in the form of nano- and/or microparticles.

As mentioned, the absorbent body is a sponge body. A sponge body particularly advantageously has a larger absorbent surface area.

The absorbent sponge body in a further embodiment is formed from a synthetic, preferably biocompatible polymer. The polymer may be a homo- or copolymer. The absorbent sponge body is preferably formed from a non-absorbable polymer, in particular selected from the group consisting of polypropylene, polyethylene, polyethylene terephthalate, polyurethane, polyvinyl alcohol, polyester, carboxymethyl cellulose, copolymers thereof, derivatives thereof, copolymers thereof and mixtures, in particular blends, thereof. The absorbent sponge body is particularly preferably formed from polyurethane or a polyurethane derivative, in particular polyurethane ether or copolymers thereof. The polyurethane may be an aliphatic polyurethane. The polyurethane is preferably a linear, aliphatic polyurethane. The polyurethane itself may be formed from macromolecular and/or low molecular weight aliphatic diols and aliphatic diisocyanates. Primarily polycarbonates, in particular 1,6-hexanediol polycarbonate, may be envisaged as macromolecular diols. For example, 2,2,4-trimethylhexanediol, 2,4,4-trimethylhexanediol and/or 1,4-butanediol may be used as low molecular weight diols. Preferably cycloaliphatic diisocyanates, in particular 4,4-dicyclohexylmethane diisocyanate or 1,4-cyclohexyl diisocyanate, may be envisaged as aliphatic diisocyanates. The polyurethane may furthermore be produced from different diols and/or diisocyanates. Polyurethane is particularly preferred as a material for the absorbent body owing to its biocompatibility.

In an alternative embodiment, the absorbent sponge body is formed from an absorbable polymer, in particular selected from the group consisting of polylactide, polyglycolide, poly-ε-caprolactone, trimethylene carbonate, poly-para-dioxanone, hydroxybutyric acid, copolymers thereof and mixtures, in particular blends, thereof. In a further embodiment, the absorbent sponge body is a co- or terpolymer comprising at least one monomer selected from the group consisting of lactide, glycolide, ε-caprolactone, trimethylene carbonate, para-dioxanone, hydroxybutyric acid, copolymers thereof and mixtures, in particular blends, thereof.

The absorbent sponge body according to the invention comprises a drainage tube. The drainage tube is used for preferably continuous removal or discharge of liquid quantities absorbed by the absorbent sponge body.

In a further embodiment, the absorbent sponge body is connected integrally to the drainage tube. In the case of the drainage tube, distinction can generally be made between a proximal (near the body of a patient) and a distal (away from the body of a patient) tube end. The drainage tube is preferably connected to the absorbent sponge body with its proximal end, whereas the distal end of the drainage tube is free and may for example be connected to a suction or vacuum source, in particular a suction or vacuum pump. With the aid of the suction or vacuum source, a negative pressure or suction of between 400 and 900 mbar (which approximately corresponds to from 300 mmHg to 675 mmHg), in particular 500 and 800 mbar (which approximately corresponds to from 375 mmHg to 600 mmHg) may be generated. Particularly rapid cleaning of infectious body cavities, and rapid granulation in the body cavities, are therefore possible.
The vacuum source may for example be a portable vacuum pump. In this way, the patient's mobility can be maintained during the treatment. In principle, the absorbent sponge body may be provided for placement in a body cavity for several hours to several days. The absorbent sponge body is typically changed every 8 to 72 hours. The quantities of fluid removed or discharged are normally gathered in collection containers intended for this, for example canisters or vacuum bottles. The collection containers are generally connected upstream of a suction or vacuum source and are in contact with it through suitable connection tubes. In order to avoid contaminating the suction or vacuum source, a sterile filter may be provided between the collection containers and the suction or vacuum source.

In the context of the invention, the absorbent sponge body may furthermore be formed integrally on the drainage tube. For example, the absorbent sponge body may be adhesively bonded, stitched or welded to the drainage tube and/or expansion-moulded onto the drainage tube. In general, at least a part of the drainage tube will be enclosed or encapsulated by the absorbent sponge body. For example, to this end the absorbent sponge body has an essentially cylindrically shaped through-channel which is preferably formed extending in the longitudinal direction of the absorbent sponge body. The through-channel may furthermore be formed by cross- or star-shaped stamping (without material being removed). The through-channel usually extends centrally through the absorbent sponge body. Expediently, the through-channel has a diameter which is adapted to the diameter of the drainage tube. That part of the drainage tube which is encapsulated by the absorbent sponge body conventionally has openings. A uniform negative pressure can therefore particularly advantageously be generated on the entire absorbent sponge body. The openings also permit more rapid and more efficient discharge of the wound fluids absorbed by the absorbent sponge body. The drainage tube itself is preferably formed from a liquid-impermeable, in particular air-impermeable material, in particular a polymer. For example, the drainage tube may be a plastic or synthetic tube. Suitable materials for the drainage tube are for example polyethylene, polypropylene, polyvinyl chloride or polyurethane.

In another possible embodiment, the absorbent sponge body comprises a drainage tube which is a liquid-tightly encapsulated section, in particular a tubular projection, of the absorbent sponge body. The encapsulation may be formed as a film, in particular an adhesive film. For example, the encapsulation may be formed from a hot-melt adhesive. Both absorbable and non-absorbable materials may be envisaged as a hot-melt adhesive. Absorbable hot-melt adhesives may for example be selected from the group consisting of polyglycolides, polylactides, polydioxanones, polycaprolactones and copolymers thereof. Polymer mixtures or polymer blends may also be envisaged.

In another embodiment, the absorbent sponge body comprises a flushing or rinsing tube. The absorbent sponge body preferably comprises both a drainage tube and a flushing tube. With the aid of the flushing tube, the absorbent sponge body can be flushed. The flushing or rinsing liquid used may for example be a sodium chloride solution, buffer solution, anti-inflammatory, odour-inhibiting and/or antimicrobial solution.

According to the invention, the film may furthermore cover not only the absorbent sponge body but also the drainage and/or a flushing tube according to one of the embodiments described above.

The absorbent sponge body according to the invention may furthermore be provided in a sterilized form and, in particular, in a packaged form. In this regard, in principle all sterilizing and packaging methods familiar to the person skilled in the art may be envisaged.

According to a particularly preferred embodiment, the absorbent sponge body according to the invention is provided as a drainage article or drainage product, preferably for discharging pathological fluid accumulations from human and/or animal body cavities.

Other features and details of the invention may be found in the following description of preferred embodiments in the form of figure descriptions. The figures are hereby made part of the content of this description by explicit reference. Individual features may respectively be implemented on their own, or several together in combination with one another. The figures, including the associated figure descriptions merely serve to explain the present invention without in any way restricting it thereto.

The following are shown schematically in the figures:
- Figure 1:: an absorbent body (before and after the wrapping is removed),
- Figure 2:: an embodiment of an absorbent body according to the invention,
- Figure 3:: another embodiment of an absorbent body according to the invention,
- Figures 4a,b:: another embodiment of an absorbent body.

### Description of the Figures

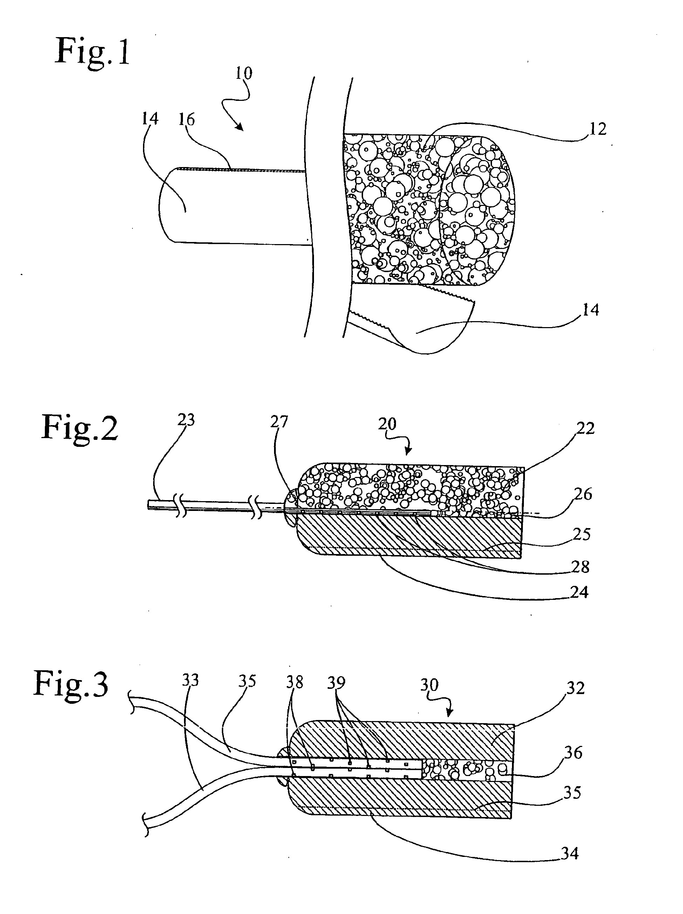

Figure 1 shows an absorbent body 10 according to the invention. It is formed as an open-pore sponge body 12 and is held in a compressed form by means of a wrapping 14. The sponge body 12 has an essentially cylindrical or tubular configuration. The wrapping 14 is formed as a film and has perforations 16 as seen in the longitudinal direction. The perforations 16 make it easier to remove the film 14 after the absorbent body 10 has been placed optimally in a body cavity to be treated. After the film 14 is removed, the sponge body 12 can unfold or expand again to its original size and shape, and in this state is ready to absorb wound fluids or wound secretions.

Figure 2 shows an absorbent body 20 according to the invention. It is formed as a cylindrically shaped or tubular sponge body 22 that is held together in a compressed form by the aid of a wrapping (represented by shading). The absorbent body 20 furthermore comprises a drainage tube 23. The drainage tube 23 is connected in one piece or integrally to the sponge body 22. The wrapping 24 itself is formed as a film which has a perforation line 25. The sponge body 22 furthermore has a cylindrical through-channel 26, which extends approximately centrally in the longitudinal direction of the sponge body 22. The drainage tube 23 projects partially into the through-channel 26. In order to fasten the sponge body 22 to the drainage tube 23, the sponge body 22 is stitched with a thread 27 or adhesively bonded at its end facing the drainage tube 23. After the absorbent body 20 represented schematically in Figure 2 has been positioned, the film 24 is removed by pulling. The removal of the film 24 is assisted or facilitated by the perforations 25 which have been formed. After the film 24 has been removed, the sponge body 22 expands to its original size and shape and is therefore available with its original volume to absorb wound secretions. With the aid of the drainage tube 23, the wound secretions absorbed by the sponge body 22 can be removed or discharged. This is usually done by applying a negative pressure to the free or distal end of the drainage tube 23. In general, for this purpose the drainage tube 23 is connected to a suction pump. The drainage tube 23 additionally comprises openings 28 in its wall, so that a maximally uniform negative pressure can be generated on the sponge body 22 and rapid drainage of the sponge body 22 and therefore the body cavities to be treated is thus possible. The openings 28 are expediently formed only in that section of the drainage tube 23 which projects into the sponge body 22.

The absorbent body 30 according to the invention, schematically represented in Figure 3, comprises a sponge body 32 which is held together in a compressed form by a wrapping 34 (represented by shading) formed as a film. The film 34 has a perforation line 35, which allows it to be removed more easily under tensile stress. Besides a drainage tube 33, the sponge body 32 also has a flushing tube 35. The tubes 33 and 35 project together into a cylindrical through-channel 36 of the sponge body 32. The tubes 33 and 35 furthermore comprise openings 38 and 39 in those sections which are covered or enclosed by the sponge body 32. With respect to other features, reference is made to the figure description for Figure 2.

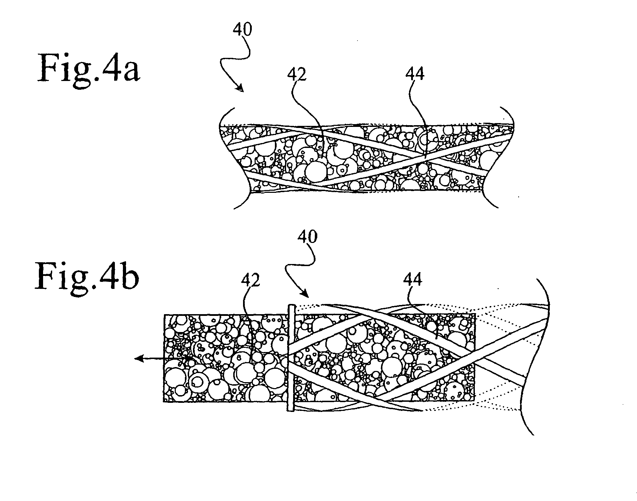

Figures 4a and 4b schematically show another embodiment of an absorbent body 40. In this embodiment, a sponge body 42 is contained in a so-called extension sleeve 44 (finger extender). The extension sleeve 44 is preferably a braided tube or round braid, whose internal space (lumen) narrows under tensile stress of the braid in the longitudinal direction and whose internal space widens when the braid is compressed in the longitudinal direction. The braid itself is preferably provided as a loose and in particular wide-meshed braid. The braid may in principle be formed from wire or bast fibres. Particularly suitable materials are polypropylene or PTFE, which prevent undesired growth of the sponge body 42 with the walls of a body cavity owing to their hydrophobic character. In order to place the absorbent body 40 in a body cavity, the extension sleeve 44 is generally stretched. After placement in a body cavity, the extension sleeve 44 is compressed. If, however, the absorbent body 40 cannot be placed optimally inside a body cavity at the first attempt, then compression of the extension sleeve 44 is not carried out until after optimal placement of the absorbent body 40. In this way, the absorbent body 40 can be re-placed, optionally several times, inside a body cavity without significant resistance. Once the absorbent body 40 is in an optimal position in therapeutic terms, a vacuum or negative pressure can be applied through a drainage tube (as represented in Figures 2 and 3) so that the walls of the wound cavity are sucked onto the sponge body 42. The meshes of the braided tube 44 allow sufficient contact with the surface of the sponge body 42 for drainage of the body cavity. If the absorbent body 40 is intended to be replaced or the drainage is completed, a tension is exerted in the longitudinal direction of the braided tube 44 so that the sponge body 42 is compressed again and can thereby be removed from the body cavity without significant resistance.

## Claims

1. Absorbent medical sponge body (20; 30) for removing wound fluids from human and/or animal body cavities, comprising a film (24; 34) for the absorbent sponge body (20; 30), wherein the absorbent sponge body (20; 30) is contained in the film (24; 34) and is held together in a compressed form by it, **characterized in that** the absorbent sponge body (20; 30) comprises a drainage tube (23; 33).

2. Absorbent medical sponge body (20; 30) according to claim 1, **characterized in that** the film (24; 34) comprises means for removing it.

3. Absorbent medical sponge body (20; 30) according to claim 1 or 2, **characterized in that** the film (24; 34) comprises at least one perforation.

4. Absorbent medical sponge body (20; 30) according to one of the preceding claims, **characterized in that** the film (24; 34) is formed from a polymer selected from the group consisting of silicone, silicone rubber, polypropylene, polyethylene, polyester, polyamides, derivatives thereof, copolymers thereof and mixtures, in particular blends, thereof.

5. Absorbent medical sponge body (20; 30) according to one of the preceding claims, **characterized in that** the film (24; 34) is provided as a hollow body.

6. Absorbent medical sponge body (20; 30) according to Claim 5, **characterized in that** the film (24; 34) is provided as a sleeve or tube.

7. Absorbent medical sponge body (20; 30) according claim 5, **characterized in that** the hollow body comprises a lumen which contracts under tensile stress in the longitudinal direction of the hollow body and is enlarged under compression in the longitudinal direction of the hollow body.

8. Absorbent medical sponge body (20; 30) according to one of the preceding claims, **characterized in that** the absorbent sponge body (20; 30) comprises pores with a diameter of between 100 and 1500 µm.

9. Absorbent medical sponge body (20; 30) according to one of the preceding claims, **characterized in that** the absorbent sponge body (20; 30) is formed from a non-absorbable polymer selected from the group consisting of polypropylene, polyethylene, polyethylene terephthalate, polyurethane, silicone, polyvinyl alcohol, polyester, derivatives thereof, copolymers thereof and mixtures thereof.

10. Absorbent medical sponge body (20; 30) according to one of the claims 1 to 8, **characterized in that** the absorbent sponge body (20; 30) is formed from an absorbable polymer selected from the group consisting of polylactide, polyglycolide, poly-ε-caprolactone, trimethylene carbonate, poly-para-dioxanone, hydroxybutyric acid, copolymers thereof and mixtures thereof.

11. Absorbent medical sponge body (20; 30) according to one of the preceding claims, **characterized in that** the absorbent sponge body (20; 30) comprises a flushing tube (35).

12. Absorbent medical sponge body (20; 30) according to one of the preceding claims, **characterized in that** the absorbent sponge body (10; 20; 30; 40) is provided as a drainage article.

## Patentansprüche

1. Saugfähiger medizinischer Schwammkörper (20; 30) zur Entfernung von Wundflüssigkeiten aus menschlichen und/oder tierischen Körperhöhlen, umfassend eine Umhüllung (24; 34) für den saugfähigen Schwammkörper (20; 30), wobei der saugfähige Schwammkörper (20; 30) in der Umhüllung (24; 34) enthalten und durch sie in einer komprimierten Form zusammengehalten ist, **dadurch gekennzeichnet, dass** der saugfähige Schwammkörper (20; 30) einen Drainageschlauch (23; 33) umfasst.

2. Saugfähiger medizinischer Schwammkörper (20; 30) nach Anspruch 1, **dadurch gekennzeichnet, dass** die Umhüllung (24; 34) Mittel zu ihrer Entfernung aufweist.

3. Saugfähiger medizinischer Schwammkörper (20; 30) nach Anspruch 1 oder 2, **dadurch gekennzeichnet, dass** die Umhüllung (24; 34) zumindest eine Perforation aufweist.

4. Saugfähiger medizinischer Schwammkörper (20; 30) nach einem der vorhergehenden Ansprüche, **dadurch gekennzeichnet, dass** die Umhüllung (24; 34) aus einem Polymer ausgewählt aus der Gruppe bestehend aus Silikon, Silikonkautschuk, Polypropylen, Polyethylen, Polyester, Polyamiden, Derivaten davon, Copolymeren davon und Mischungen, insbesondere Blends, davon gebildet ist.

5. Saugfähiger medizinischer Schwammkörper (20; 30) nach einem der vorhergehenden Ansprüche, **dadurch gekennzeichnet, dass** die Umhüllung (24; 34) als Hohlkörper vorliegt.

6. Saugfähiger medizinischer Schwammkörper (20; 30) nach Anspruch 5, **dadurch gekennzeichnet, dass** die Umhüllung (24; 34) als Hülse oder Schlauch vorliegt.

7. Saugfähiger medizinischer Schwammkörper (20; 30) nach Anspruch 5, **dadurch gekennzeichnet, dass** der Hohlkörper ein Lumen aufweist, welches sich bei Zugbelastung in Längsrichtung des Hohlkörpers verengt und bei Stauchung in Längsrichtung des Hohlkörpers vergrößert.

8. Saugfähiger medizinischer Schwammkörper (20; 30) nach einem der vorhergehenden Ansprüche, **dadurch gekennzeichnet, dass** der saugfähige Schwammkörper (20; 30) Poren mit einem Durchmesser zwischen 100 und 1500 µm aufweist.

9. Saugfähiger medizinischer Schwammkörper (20; 30) nach einem der vorhergehenden Ansprüche, **dadurch gekennzeichnet, dass** der saugfähige Schwammkörper (20; 30) aus einem nicht resorbierbaren Polymer ausgewählt aus der Gruppe bestehend aus Polypropylen, Polyethylen, Polyethylenterephthalat, Polyurethan, Silikon, Polyvinylalkohol, Polyester, Derivaten davon, Copolymeren davon und Mischungen davon gebildet ist.

10. Saugfähiger medizinischer Schwammkörper (20; 30) nach einem der Ansprüche 1 bis 8, **dadurch gekennzeichnet, dass** der saugfähige Schwammkörper (20; 30) aus einem resorbierbaren Polymer ausgewählt aus der Gruppe bestehend aus Polylactid, Polyglykolid, Poly-ε-caprolacton, Trimethylencarbonat, Poly-para-dioxanon, Hydroxybuttersäure, Copolymeren davon und Mischungen davon gebildet ist.

11. Saugfähiger medizinischer Schwammkörper (20; 30) nach einem der vorhergehenden Ansprüche, **dadurch gekennzeichnet, dass** der saugfähige Schwammkörper (20; 30) einen Spülschlauch (35) aufweist.

12. Saugfähiger medizinischer Schwammkörper (20; 30) nach einem der vorhergehenden Ansprüche, **dadurch gekennzeichnet, dass** der saugfähige Schwammkörper (20; 30) als Drainageartikel vorliegt.

## Revendications

1. Corps d'éponge médicale absorbant (20; 30) pour extraire les fluides de blessure de cavités corporelles humaines et/ou animales, comprenant un film (24; 34) pour le corps d'éponge absorbant (20; 30), dans lequel le corps d'éponge absorbant (20; 30) est contenu dans le film (24; 34) et est maintenu sous une forme comprimée par celui-ci, **caractérisé en ce que** le corps d'éponge absorbant (20; 30) comprend un tube de drainage (23; 33).

2. Corps d'éponge médicale absorbant (20; 30) selon la revendication 1, **caractérisé en ce que** le film (24; 34) comprend des moyens pour enlever celui-ci.

3. Corps d'éponge médicale absorbant (20; 30) selon la revendication 1 ou 2, **caractérisé en ce que** le film (24; 34) comporte au moins une perforation.

4. Corps d'éponge médicale absorbant (20; 30) selon l'une quelconque des revendications précédentes, **caractérisé en ce que** le film (24; 34) est formé à partir d'un polymère qui est sélectionné dans le groupe comprenant le silicone, le caoutchouc silicone, le polypropylène, le polyéthylène, le polyester, des polyamides, des dérivés de ceux-ci, des copolymères de ceux-ci et des compositions, en particulier des mélanges, de ceux-ci.

5. Corps d'éponge médicale absorbant (20; 30) selon l'une quelconque des revendications précédentes, **caractérisé en ce que** le film (24; 34) est fourni sous la forme d'un corps creux.

6. Corps d'éponge médicale absorbant (20; 30) selon la revendication 5, **caractérisé en ce que** le film (24; 34) est fourni sous la forme d'un manchon ou d'un tube.

7. Corps d'éponge médicale absorbant (20; 30) selon la revendication 5, **caractérisé en ce que** le corps creux comprend une lumière qui se contracte sous une contrainte de traction dans la direction longitudinale du corps creux, et qui est agrandie en cas de compression dans la direction longitudinale du corps creux.

8. Corps d'éponge médicale absorbant (20; 30) selon l'une quelconque des revendications précédentes, **caractérisé en ce que** le corps d'éponge absorbant (20; 30) comporte des pores dont le diamètre est compris entre 100 µm et 1500 µm.

9. Corps d'éponge médicale absorbant (20; 30) selon l'une quelconque des revendications précédentes, **caractérisé en ce que** le corps d'éponge absorbant (20; 30) est formé à partir d'un polymère non absorbable qui est sélectionné dans le groupe comprenant le polypropylène, le polyéthylène, le polyéthylène téréphtalate, le polyuréthane, le silicone, l'alcool polyvinylique, le polyester, des dérivés de ceux-ci, des copolymères de ceux-ci et des mélanges de ceux-ci.

10. Corps d'éponge médicale absorbant (20; 30) selon l'une quelconque des revendications 1 à 8, **caractérisé en ce que** le corps d'éponge absorbant (20; 30) est formé à partir d'un polymère absorbable qui est sélectionné dans le groupe comprenant le polylactide, le polyglycolide, le poly-ε-caprolactone, le carbonate de triméthylène, le poly-para-dioxanone, l'acide hydroxybutyrique, des copolymères de ceux-ci et des mélanges de ceux-ci.

11. Corps d'éponge médicale absorbant (20; 30) selon l'une quelconque des revendications précédentes, **caractérisé en ce que** le corps d'éponge absorbant (20; 30) comprend un tube d'injection (35).

12. Corps d'éponge médicale absorbant (20; 30) selon l'une quelconque des revendications précédentes, **caractérisé en ce que** le corps d'éponge absorbant (10; 20; 30; 40) est fourni sous la forme d'un produit de drainage.
